# EUROPEAN PATENT APPLICATION

(11) **EP 4 624 570 A1**
(43) Date of publication of application: **01.10.2025**
(21) Application number: 23894903.6
(22) Date of filing: 14.11.2023
(51) Int. Cl.: C12N 5/0783, C07K 14/765, A61K 35/17, A61P 37/06

(54) **METHOD FOR MASS PRODUCTION OF IMMUNOSUPPRESSIVE T CELLS USING HUMAN SERUM ALBUMIN**

(30) Priority: 23.11.2022 KR 20220158465; 13.11.2023 KR 20230156451
(71) Applicant: The Catholic University Of Korea Industry-Academic Cooperation Foundation, Seocho-gu Seoul 06591 (KR)
(72) Inventor: KIM, Jeong A, Seoul 06647 (KR)
(74) Representative: Global IP Europe Patentanwaltskanzlei
(86) International application number: PCT/KR2023/018206
(87) International publication number: WO 2024/111974

(57) **Abstract**

The present disclosure relates to a method for the mass production of immunosuppressive T cells using human serum albumin. According to the present disclosure, when peripheral blood stem cells (PBSCs) obtained from individuals who have received granulocyte colony stimulating factor (G-CSF), are cultured in a medium comprising human serum albumin (HSA) or HSA and mercaptoethanol, immunosuppressive T cells having an immunosuppressive effect can be produced in large quantities, it was confirmed that the immunosuppressive T cells show excellent treatment effects against acute graft-versus-host disease, and thus, the present disclosure is expected to be useful in the treatment of immune diseases such as acute graft-versus-host disease.

## Description

### [Technical Field]

The present disclosure relates to a method for the mass production of immunosuppressive T cells using human serum albumin.

### [Background Art]

Acute graft-versus-host disease (aGVHD) is an immune response that occurs due to a mismatch in the major histocompatibility complex (MHC) between transplanted immune cells and cells in a patient's body after transplantation of allogeneic blood stem cells. Approximately 20% of patients with graft-versus-host disease do not respond to commonly used steroid treatment and require long-term use of various types of immunosuppressants, which result in a decreased quality of life due to side effects and ultimately lead to death from infection. To prevent organ damage and improve survival rate due to graft-versus-host disease, cell therapy that may correct the imbalance between immune cells is the most ideal rather than using various immunosuppressants in combination. However, mesenchymal stem cells (MSCs), which have been currently used as a cellular therapeutic agent, are not widely used in clinical practice because their therapeutic effects are temporary and minimal.

Meanwhile, according to recent research reports, T lymphocytes expressing surface antigens such as PD1, TIM3, TIGIT, and LAG3 on the cell surface are known to have an effect of suppressing immune responses, unlike other T lymphocytes. However, since CD3⁺PD1⁺TIM3⁺ cells having an immunosuppressive effect exist in very small quantities in the blood (3.3 ± 2.9% in CD3⁺ lymphocytes), there is a problem that it is difficult to be used as a cellular therapeutic agent in clinical trials.

Accordingly, as a result of research for the mass production of T cells having an excellent immunosuppressive effect, it was confirmed that when CD3⁺ lymphocytes were cultured in a medium containing human serum albumin (HSA) and mercaptoethanol, immunosuppressive T lymphocytes such as CD3⁺PD1⁺TIM3⁺ lymphocytes and CD3⁺PD1⁺TIM3⁻ lymphocytes were significantly increased by surface antigens, and then the present disclosure was completed.

### [Disclosure]

### [Technical Problem]

An object of the present disclosure is to provide a method for the mass production of immunosuppressive T cells.

Another object of the present disclosure is to provide a method for inducing T cells *in vitro* into T cells having at least one phenotype selected from the group consisting of CD3⁺PDI⁺TIM3⁺, CD3⁺PD1⁺TIM3⁻ and CD3⁺PDI⁻TIM3⁻.

Yet another object of the present disclosure is to provide a method for inducing T cells *in vitro* into T cells having a phenotype of CD3⁺PD1⁺TIM3⁺.

Still another object of the present disclosure is to provide a medium composition for the mass production of immunosuppressive T cells.

Still another object of the present disclosure is to provide a kit for the mass production of immunosuppressive T cells.

Still another object of the present disclosure is to provide a medium composition for the proliferation of immunosuppressive T cells having at least one phenotype selected from the group consisting of CD3⁺PD1⁺TIM3⁺, CD3⁺PD1⁺TIM3⁻ and CD3⁺PD1⁻TIM3⁻.

Still another object of the present disclosure is to provide a medium composition for the proliferation of immunosuppressive T cells having a phenotype of CD3⁺PD1⁺TIM3⁺.

Still another object of the present disclosure is to provide a kit for the proliferation of immunosuppressive T cells having at least one phenotype selected from the group consisting of CD3⁺PDI⁺TIM3⁺, CD3⁺PDI⁺TIM3⁻ and CD3⁺PDI⁻TIM3⁻.

Still another object of the present disclosure is to provide a kit for the proliferation of immunosuppressive T cells having a phenotype of CD3⁺PD1⁺TIM3⁺.

Still another object of the present disclosure is to provide a pharmaceutical composition for preventing or treating autoimmune diseases.

Still another object of the present disclosure is to provide a cell therapeutic agent composition for preventing or treating autoimmune diseases.

Still another object of the present disclosure is to provide a method for preventing or treating autoimmune diseases.

### [Technical Solution]

An aspect of the present disclosure provides a method for the mass production of immunosuppressive T cells, including the following steps: sorting CD3⁺ cells from a sample isolated from an individual; and culturing the CD3⁺ cells in a medium containing human serum albumin (HSA).

Another aspect of the present disclosure provides a method for the mass production of immunosuppressive T cells, including the following steps: sorting CD3⁺ cells from a sample isolated from an individual administered with a granulocyte colony stimulating factor (G-CSF); and culturing the CD3⁺ cells in a medium containing human serum albumin (HSA) and mercaptoethanol for 1 to 7 days.

Yet another aspect of the present disclosure provides a method for inducing T cells *in vitro* into T cells having at least one phenotype selected from the group consisting of CD3⁺PD1⁺TIM3⁺, CD3⁺PD1⁺TIM3⁻ and CD3⁺PD1⁻TIM3⁻, the method including the following steps: sorting CD3⁺ cells from a sample isolated from an individual administered with a granulocyte colony stimulating factor (G-CSF); and culturing the CD3⁺ cells in a medium containing human serum albumin (HSA) and mercaptoethanol for 1 to 3 days.

Still another aspect of the present disclosure provides a method for inducing T cells *in vitro* into T cells having a phenotype of CD3⁺PD1⁺TIM3⁺, the method including the following steps: sorting CD3⁺ cells from a sample isolated from an individual administered with a granulocyte colony stimulating factor (G-CSF); and culturing the CD3⁺ cells in a medium containing human serum albumin (HSA) and mercaptoethanol for 4 to 7 days.

Still another aspect of the present disclosure provides a medium composition for the mass production of immunosuppressive T cells, including human serum albumin (HSA).

Still another aspect of the present disclosure provides a kit for the mass production of immunosuppressive T cells, including a granulocyte colony stimulating factor (G-CSF) and human serum albumin (HSA).

Still another aspect of the present disclosure provides a medium composition for the proliferation of immunosuppressive T cells having at least one phenotype selected from the group consisting of CD3+PD1+TIM3+, CD3+PD1+TIM3- and CD3+PD1-TIM3, including human serum albumin (HSA) and characterized by culturing peripheral blood stem cells (PBSCs) for 1 to 3 days.

Still another aspect of the present disclosure provides a medium composition for the proliferation of immunosuppressive T cells having a phenotype of CD3⁺PDI⁺TIM3⁺, including human serum albumin (HSA) and characterized by culturing peripheral blood stem cells (PBSCs) for 4 to 7 days.

Still another aspect of the present disclosure provides a kit for the proliferation of immunosuppressive T cells having at least one phenotype selected from the group consisting of CD3⁺PD1⁺TIM3⁺, CD3⁺PD1⁺TIM3⁻, and CD3⁺PD1⁻TIM3⁻, including a granulocyte colony stimulating factor (G-CSF) and human serum albumin (HSA) and characterized by culturing peripheral blood stem cells (PBSCs) for 1 to 3 days.

Still another aspect of the present disclosure provides a kit for the proliferation of immunosuppressive T cells having a phenotype of CD3⁺PD1⁺TIM3⁺, including a granulocyte colony stimulating factor (G-CSF) and human serum albumin (HSA) and characterized by culturing peripheral blood stem cells (PBSCs) for 4 to 7 days.

Still another aspect of the present disclosure provides a pharmaceutical composition for preventing or treating autoimmune diseases, including immunosuppressive T cells prepared through the method for the mass production of immunosuppressive T cells according to the present disclosure.

Still another aspect of the present disclosure provides a cell therapeutic agent composition for preventing or treating autoimmune diseases, including immunosuppressive T cells having a phenotype of CD3⁺PD1⁺TIM3⁺ prepared through the method for the mass production of immunosuppressive T cells according to the present disclosure.

Still another aspect of the present disclosure provides a method for preventing or treating autoimmune diseases, including administering, to an individual, immunosuppressive T cells prepared through the method for the mass production of immunosuppressive T cells according to the present disclosure.

### [Advantageous Effects]

According to the present disclosure, when peripheral blood stem cells (PBSCs) obtained from individuals who have received granulocyte colony stimulating factor (G-CSF), are cultured in a medium comprising human serum albumin (HSA) or HSA and mercaptoethanol, immunosuppressive T cells having an immunosuppressive effect can be produced in large quantities, and it was confirmed that the immunosuppressive T cells show excellent treatment effects against acute graft-versus-host disease.

### [Description of Drawings]

FIG. 1 shows results of confirming the cell count, when CD3⁺ lymphocytes isolated from G-CSF mobilized peripheral blood stem cells (G-PBSC) are cultured in a medium containing human serum albumin (HSA) or a medium containing HSA and mercaptoethanol.
FIG. 2 shows results of confirming the number of CD3⁺ lymphocytes (CD3⁺PBSC) isolated from the blood of a normal individual, CD3⁺G-PBSC (D0 CD3⁺G-PBSC) before culture, and CD3⁺PD1⁺, CD3⁺PD1⁺TIM3⁺, CD3⁺PD1⁺LAG3⁺ and CD3⁺PD1⁺TIGIT⁺ lymphocytes in CD3⁺G-PBSC (D4 CD3⁺G-PBSC) cultured for 4 days using a method according to the present disclosure.
FIG. 3 shows results of a mixed lymphocyte reaction measured in CD3⁺PD1⁺TIM3⁺ and CD3⁺PD1⁻TIM3⁻ lymphocytes isolated from CD3⁺G-PBSC cultured for 4 days by the method according to the present disclosure.
FIG. 4 is a diagram of confirming TIGIT and LAG3 surface antigens expressed in CD3⁺PD1⁺TIM3⁺ lymphocytes cultured for 4 days using the method according to the present disclosure.
FIG. 5 is a diagram of confirming a mixed lymphocyte reaction measured in CD3⁺G-PBSC (control group) before culture and CD3⁺G-PBSC cultured for 2 or 4 days using the method according to the present disclosure.
FIG. 6 is a diagram showing the number of CD3⁺G-PBSC (control group) before culture and CD3⁺PD1⁺TIM3⁺, CD3⁺PD1⁺TIM3⁻, and CD3⁺PD1⁻TIM3⁻ lymphocytes in CD3⁺G-PBSC cells cultured for 2 or 4 days using the method according to the present disclosure.
FIG. 7 shows results of measuring a mixed lymphocyte reaction in each lymphocyte after isolating CD3⁺PD1⁺TIM3⁺, CD3⁺PD1⁻TIM3⁻, and CD3⁺PD1⁺TIM3⁻ lymphocytes from CD3⁺G-PBSC cultured for 2 or 4 days by the method according to the present disclosure, and results of measuring the mixed lymphocyte reaction after treating the isolated lymphocytes with anti-PD1 mAb or anti-Tim3 mAb according to the expression of cell surface antigens.
FIG. 8 shows results of measuring the amount of CD3⁺PD1⁺TIM3⁺ lymphocytes differentiated from CD3⁺PD1⁻TIM3⁻ and CD3⁺PD1⁺TIM3⁻ lymphocytes, after isolating only CD3⁺PD1⁻TIM3⁻ and CD3⁺PD1⁺TIM3⁻ lymphocytes from CD3⁺G-PBSC before culture and culturing the lymphocytes for 4 days using the method of the present disclosure.
FIG. 9 is a diagram showing results of evaluating a therapeutic effect of CD3⁺G-PBSC cells cultured for 4 days by the method of the present disclosure, using a xenograft aGVHD mouse model (NOD-scid IL-2Rγ null).
FIG. 10 is a diagram showing results of evaluating a therapeutic effect of CD3⁺G-PBSC cells cultured for 2 days by the method of the present disclosure, using a xenograft aGVHD mouse model (NOD-scid IL-2Rγ null).

### [Modes]

Hereinafter, embodiments of the present disclosure will be described in detail with reference to the accompanying drawings. In the following description, detailed descriptions of techniques well-known to those skilled in the art may be omitted. Further, in describing the present disclosure, the detailed description of associated known functions or constitutions will be omitted if it is determined to unnecessarily make the gist of the present disclosure unclear. In addition, terminologies used herein are terminologies used to properly express preferred exemplary embodiments of the present disclosure, which may vary according to a user, an operator's intention, or customs in the art to which the present disclosure pertains.

Accordingly, definitions of the terminologies need to be described based on contents throughout this specification. Throughout this specification, unless explicitly described to the contrary, when a certain part "comprises" a certain component, it will be understood to imply the inclusion of stated elements, not the exclusion of any other elements.

The present disclosure provides a method for the mass production of immunosuppressive T cells, including the following steps: sorting CD3⁺ cells from a sample isolated from an individual; and culturing the CD3⁺ cells in a medium containing human serum albumin (HSA).

In the present disclosure, the term "CD3" refers to a T cell co-receptor, a molecule involved in the activation of cytotoxic T cells and helper T cells.

In the present disclosure, the term "human serum albumin" refers to a water-soluble protein that constitutes most of human serum proteins and may regulate the osmotic pressure of blood and transport substances through the blood.

In the present disclosure, the sample may be at least one selected from the group consisting of blood, tissue, cells, plasma, feces, urine, and semen, but is not limited thereto. Preferably, the sample may be at least one selected from the group consisting of blood and peripheral blood stem cells (PBSCs), and more preferably PBSCs.

In the present disclosure, the term "peripheral blood stem cell (PBSC)" refers to a blood stem cell existing in peripheral blood vessels. The blood stem cells are stem cells that produce blood, and refer to stem cells that produce all cells in the blood, including red blood cells, white blood cells, platelets, T cells, and the like.

In addition, the individual may be a healthy allogeneic individual. Preferably, the individual may be a healthy allogeneic individual with an individual to be administered with the immunosuppressive T cells of the present disclosure.

In addition, the individual is characterized by the mobilization of CD3⁺ cells into the peripheral blood. The mobilization may mean that CD3⁺ cells move into the peripheral blood. In the present disclosure, the mobilization of the CD3⁺ cells into the peripheral blood may be performed using any method known in the art without limitation. In one embodiment of the present disclosure, a leucocyte growth factor was administered to the individual for the mobilization.

Therefore, the individual may be an individual administered with the leucocyte growth factor, and the leucocyte growth factor may be a granulocyte colony stimulating factor (G-CSF).

In the present disclosure, the term "granulocyte colony stimulating factor (G-CSF)" is a type of glycoprotein that promotes the proliferation and differentiation of blood stem cells and enhances the survival capacity of neutrophils, and thus can be used to prevent and treat neutropenia.

Meanwhile, according to one embodiment of the present disclosure, in the method for the mass production of immunosuppressive T cells of the present disclosure, it was confirmed that when CD3⁺ lymphocytes were cultured in a medium containing HSA, the number of cells increased 0.4 ± 0.1 times compared to before culturing. In addition, when the cells were cultured in a medium containing HSA and mercaptoethanol, it was confirmed that the number of cells increased 1.0 ± 0.6 times compared to before culture, so that the yield of CD3⁺ lymphocytes was significantly increased.

Therefore, in the method for the mass production of the immunosuppressive T cells of the present disclosure, the medium may further include mercaptoethanol.

In the present disclosure, the term "mercaptoethanol" refers to a compound that may reduce disulfide bonds and remove hydroxyl radicals, and thus is used as a biological reducing agent and an antioxidant.

The concentration of the mercaptoethanol may be 1 µM to 200 µM, but is not limited thereto. Preferably, the concentration of the mercaptoethanol may be 20 µM to 100 µM, and more preferably 30 µM to 70 µM, but is not limited thereto. More preferably, the concentration of the mercaptoethanol may be 30 µM to 60 µM, 30 µM to 50 µM, 40 µM to 70 µM, 40 µM to 60 µM, 40 µM to 50 µM, 50 µM to 70 µM, or 50 µM to 60 µM, but is not limited thereto.

In addition, the concentration of the human serum albumin (HSA) may be 1 wt% to 10 wt%, but is not limited thereto. Preferably, the concentration of the human serum albumin may be 1 wt% to 8 wt%, and more preferably 2 wt% to 8 wt%, but is not limited thereto. More preferably, the concentration of the human serum albumin may be 2 wt% to 7 wt%, 2 wt% to 6 wt%, 2 wt% to 5 wt%, 3 wt% to 8 wt%, 3 wt% to 7 wt%, 3 wt% to 6 wt%, 3 wt% to 5 wt%, 4 wt% to 8 wt%, 4 wt% to 7 wt%, 4 wt% to 6 wt%, 4 wt% to 5 wt%, 5 wt% to 8 wt%, 5 wt% to 7 wt% or 5 wt% to 6 wt%, but is not limited thereto.

The human serum albumin is an allogeneic protein rather than a heterologous protein such as fetal bovine serum (FBS), making it safer for use as a cellular therapeutic agent in clinical trials. In addition, when the concentration of the human serum albumin is 1 wt% to 10 wt%, the production yield of immunosuppressive T cells may be significantly increased, and thus can be useful in the mass production of immunosuppressive T cells.

Meanwhile, according to another embodiment of the present disclosure, in the peripheral blood of a normal individual, CD3⁺PD1⁺TIM3⁺ lymphocytes were measured to be very low at 2 ± 1 per 1 × 10³ CD3⁺G-PBSC cells. However, when CD3⁺G-PBSCs were collected after G-CSF was injected subcutaneously for 5 days in a normal individual and cultured for 4 days in a medium containing 5% HSA and mercaptoethanol, the number of CD3⁺PD1⁺TIM3⁺ lymphocytes per 1 × 10³ CD3⁺ cells was measured to be 865 ± 88. That is, it was confirmed through the method according to the present disclosure that the number of CD3⁺PD1⁺TIM3⁺ lymphocytes may be increased by 433 times compared to peripheral blood, and by about 13 times or more compared to before culture.

In addition, according to another embodiment of the present disclosure, when CD3⁺G-PBSC cells were cultured in a medium containing 5% HSA and mercaptoethanol, the number of CD3⁺PD1⁺TIM3⁺ cells increased as the culture time elapsed. The number of CD3⁺PD1⁺TIM3⁺ cells before culture was 68 ± 8 per 1 × 10³ CD3⁺G-PBSCs, but the number of CD3⁺PD1⁺TIM3⁺ cells increased to 212 ± 44 after 2 days of culture, and increased to 865 ± 88 after 4 days of culture. On the other hand, CD3⁺PD1⁺TIM3⁻ lymphocytes were 197 ± 52 per 1 × 10³ CD3⁺G-PBSCs before culture, but decreased to 172 ± 64 after 2 days of culture, and after 4 days of culture, almost all cells were lost. In addition, CD3⁺PD1⁻TIM3⁻ lymphocytes were 735 ± 105 per 1 × 10³ CD3⁺G-PBSCs before culture, but decreased to 616 ± 192 after 2 days of culture, and to 67 ± 38 after 4 days of culture.

In particular, according to another embodiment, CD3⁺PD1⁻TIM3⁻ and CD3⁺PD1⁺TIM3⁻ lymphocytes were isolated from CD3⁺G-PBSC and cultured for 4 days using the method according to the present disclosure. As a result, it was confirmed that 70.8 ± 3.1% of CD3⁺PD1⁻TIM3⁻ lymphocytes and 80.7 ± 5.2% of CD3⁺PD1⁺TIM3⁻ lymphocytes differentiated into CD3⁺PD1⁺TIM3⁺ lymphocytes. According to the results, it is estimated that CD3⁺PD1⁻TIM3⁻ lymphocytes and CD3⁺PD1⁺TIM3⁻ lymphocytes differentiate into CD3⁺PD1⁺TIM3⁺ lymphocytes as the culture time elapses.

Therefore, in the present disclosure, the term "mass production" means obtaining as many cells as the number of cells required for a therapeutic dose. The method for the mass production of immunosuppressive T cells of the present disclosure means producing cells including about 87% (86.5 ± 8.8%) of CD3⁺PD1⁺TIM3⁺ lymphocytes having an excellent immunosuppressive effect from the CD3⁺ cell fraction.

The terms "PD1" and "Tim3" are co-inhibitory molecules, and T cells expressing these factors may block excessive immune responses and maintain immune tolerance.

As used herein, the term "immune tolerance" refers to a state in which an immune response to a specific antigen is not shown. Therefore, the immunosuppressive T cells according to the present disclosure mean T cells that induce tolerance to autoantigens and suppress the proliferation of T cells, and for the purpose of the present disclosure, the immunosuppressive T cells of the present disclosure mean T cells that induce immune tolerance to autoantigens that cause autoimmune diseases, preferably graft-versus-host disease.

In the present disclosure, the culturing may be performed for 1 to 7 days, but is not limited thereto. The immunosuppressive T cells cultured for 1 to 7 days using the method according to the present disclosure are characterized by having a phenotype of CD3⁺PD1⁺TIM3⁺.

Meanwhile, when the culturing is performed for 1 to 3 days, the immunosuppressive T cells may have at least one phenotype selected from the group consisting of CD3⁺PD1⁺TIM3⁺, CD3⁺PD1⁺TIM3⁻, and CD3⁺PDI⁻TIM3⁻.

Further, the present disclosure provides a method for the mass production of immunosuppressive T cells, including the following steps: sorting CD3⁺ cells from a sample isolated from an individual administered with a granulocyte colony stimulating factor (G-CSF); and culturing the CD3⁺ cells in a medium containing human serum albumin (HSA) and mercaptoethanol for 1 to 7 days.

Further, the present disclosure provides a method for inducing T cells *in vitro* into T cells having at least one phenotype selected from the group consisting of CD3⁺PDI⁺TIM3⁺, CD3⁺PD1⁺TIM3⁻ and CD3⁺PD1⁻TIM3⁻, the method including the following steps: sorting CD3⁺ cells from a sample isolated from an individual administered with a granulocyte colony stimulating factor (G-CSF); and culturing the CD3⁺ cells in a medium containing human serum albumin (HSA) and mercaptoethanol for 1 to 3 days.

Further, the present disclosure provides a method for inducing T cells *in vitro* into T cells having a phenotype of CD3⁺PD1⁺TIM3⁺, the method including the following steps: sorting CD3⁺ cells from a sample isolated from an individual administered with a granulocyte colony stimulating factor (G-CSF); and culturing the CD3⁺ cells in a medium containing human serum albumin (HSA) and mercaptoethanol for 4 to 7 days.

The granulocyte colony stimulating factor (G-CSF) may be administered at a concentration of 1 to 50 µg/kg, but is not limited thereto. The G-CSF may be administered preferably at a concentration of 1 to 30 µg/kg, more preferably at a concentration of 5 to 20 µg/kg, but is not limited thereto. Much more preferably, the concentration of the G-CSF may be 5 to 15 µg/kg, 5 to 10 µg/kg, 6 to 20 µg/kg, 6 to 15 µg/kg, 6 to 10 µg/kg, 7 to 20 µg/kg, 7 to 15 µg/kg, 7 to 10 µg/kg, 8 to 20 µg/kg, 8 to 15 µg/kg, 8 to 10 µg/kg, 9 to 20 µg/kg, 9 to 15 µg/kg, 9 to 10 µg/kg, 10 to 20 µg/kg, or 10 to 15 µg/kg, but is not limited thereto.

Further, the present disclosure provides a medium composition for the mass production of immunosuppressive T cells, including human serum albumin (HSA).

The medium composition may further include mercaptoethanol.

The concentration of the mercaptoethanol may be 1 µM to 200 µM, but is not limited thereto. Preferably, the concentration of the mercaptoethanol may be 20 µM to 100 µM, and more preferably 30 µM to 70 µM, but is not limited thereto.

In addition, the concentration of the human serum albumin (HSA) may be 1 wt% to 10 wt%, but is not limited thereto. Preferably, the concentration of the human serum albumin may be 1 wt% to 8 wt%, and more preferably 2 wt% to 8 wt%, but is not limited thereto.

The medium composition according to the present disclosure may be used for culturing blood or peripheral blood stem cells (PBSCs), and more preferably, used for culturing PBSCs.

The immunosuppressive T cells cultured using the medium composition according to the present disclosure are characterized by having an immunophenotype of CD3⁺PD1⁺TIM3⁺.

In the present disclosure, the media mean media capable of supporting cell growth and survival *in vitro,* and include all conventional media used in the art suitable for culturing cells. The media and culture conditions may be selected according to a type of cell. The medium used for culture is preferably a cell culture minimum medium (CCMM), and generally includes a carbon source, a nitrogen source, and trace elements. As the medium, any medium generally used for culturing animal cells may be used. Preferably, a medium containing serum (e.g., fetal bovine serum, horse serum or human serum) may be used. As used herein, the medium includes, for example, RPMI series (e.g., RPMI 1640), Eagle's minimum essential medium (Eagle's MEM, Eagle), α-MEM, Iscove's MEM, 199 medium, CMRL 1066, F12, F10, Dulbecco's modification of Eagle's medium (DMEM), a mixture of DMEM and F12, Way-mouth's MB752/1, McCoy's 5A and MCDB series, but is not limited thereto. The medium may include other ingredients, for example, antioxidants or antibiotics such as penicillin, streptomycin, or gentamicin.

Further, the present disclosure provides a kit for the mass production of immunosuppressive T cells, including a granulocyte colony stimulating factor (G-CSF) and human serum albumin (HSA). The kit may further include mercaptoethanol.

The concentrations of G-CSF, HSA, and mercaptoethanol included in the kit are as described above.

The kit is not limited to those described above and may include other reagents or instruments. For example, the kit may include a culture plate for culturing target cells, and reagents required for a method (e.g., mixed lymphocyte reaction (MLR), etc.) of analyzing immunosuppressive activity, and may also have PBSCs to be cultured.

The kit according to the present disclosure may be provided in a single container containing a medium containing G-CSF and HSA or G-CSF, HSA and mercaptoethanol and all other components necessary for culture and other reagents in appropriate volumes and/or forms, or may be provided in separate containers.

The kit according to the present disclosure may include instructions describing a procedure, and the like for performing the method according to the present disclosure described above.

Further, the present disclosure provides a medium composition for the proliferation of immunosuppressive T cells having at least one phenotype selected from the group consisting of CD3⁺PD1⁺TIM3⁺, CD3⁺PD1⁺TIM3⁻, and CD3⁺PD1⁻TIM3⁻, including human serum albumin (HSA) and characterized by culturing peripheral blood stem cells (PBSCs) for 1 to 3 days.

Further, the present disclosure provides a medium composition for the proliferation of immunosuppressive T cells having a phenotype of CD3⁺PD1⁺TIM3⁺, including human serum albumin (HSA) and characterized by culturing peripheral blood stem cells (PBSCs) for 4 to 7 days.

The medium composition for the proliferation of immunosuppressive T cells according to the present disclosure may further include mercaptoethanol.

The detailed description of the medium composition, including the concentrations of HSA and mercaptoethanol, is as described above.

Further, the present disclosure provides a kit for the proliferation of immunosuppressive T cells having at least one phenotype selected from the group consisting of CD3⁺PD1⁺TIM3⁺, CD3⁺PD1⁺TIM3⁻, and CD3⁺PD1⁻TIM3⁻, including a granulocyte colony stimulating factor (G-CSF) and human serum albumin (HSA) and characterized by culturing peripheral blood stem cells (PBSCs) for 1 to 3 days.

Further, the present disclosure provides a kit for the proliferation of immunosuppressive T cells having a phenotype of CD3⁺PD1⁺TIM3⁺, including a granulocyte colony stimulating factor (G-CSF) and human serum albumin (HSA) and characterized by culturing peripheral blood stem cells (PBSCs) for 4 to 7 days.

The kit for the proliferation of immunosuppressive T cells according to the present disclosure may further include mercaptoethanol.

In the kit of the present disclosure, the detailed description including the concentrations of the G-CSF, HSA, and mercaptoethanol included is as described above.

According to another embodiment of the present disclosure, when a mixed lymphocyte reaction was performed on the entire CD3⁺G-PBSC cultured for 2 or 4 days in a medium containing 5% HSA and mercaptoethanol, it was measured that 0.5 ± 0.1% of T cells in the case of CD3⁺G-PBSC (control group) before culture did not proliferate against allogeneic monocytes, 13.9 ± 6.4% of T cells in the case of CD3⁺G-PBSC cultured for 4 days, and 43.2 ± 10.1% of T cells in the case of CD3⁺G-PBSC cultured for 2 days did not proliferate against allogeneic monocytes. From the results, CD3⁺G-PBSC cultured for 2 days showed the best immune response suppression effect, and the immune response suppression effect of these cells was superior to that of CD3⁺PD1⁺TIM3⁺ lymphocytes isolated after 4 days of culture. That is, it was confirmed that T cells cultured through the method according to the present disclosure exhibited an excellent immune response suppression effect, and the effect was far superior for T cells cultured for 2 days.

In addition, according to yet another embodiment of the present disclosure, when CD3⁺G-PBSC cells cultured according to the method of the present disclosure were injected into a xenograft aGVHD mouse model, it was confirmed that the survival of mice was significantly increased. In particular, in an experimental group injected with CD3⁺G-PBSC cultured for 2 days at a dose of 1 × 10⁷ cells/kg, 100% of target mice survived at 80 days after cell injection, which showed a significantly better therapeutic effect than CD3⁺G-PBSC cultured for 4 days. That is, it was confirmed that T cells cultured through the method according to the present disclosure exhibited an excellent graft-versus-host disease treatment effect, and the effect was far superior for T cells cultured for 2 days.

Therefore, the present disclosure provides a pharmaceutical composition for preventing or treating autoimmune diseases, including immunosuppressive T cells prepared through the method for the mass production of immunosuppressive T cells according to the present disclosure.

In the pharmaceutical composition of the present disclosure, the immunosuppressive T cells are characterized by having a phenotype of CD3⁺PD1⁺TIM3⁺. In addition, the immunosuppressive T cells may further have at least one phenotype selected from the group consisting of CD3⁺PD1⁺TIM3⁻ and CD3⁺PD1⁻TIM3⁻.

In the pharmaceutical composition according to the present disclosure, the autoimmune disease may be at least one disease selected from the group consisting of graft-versus-host disease, rheumatoid arthritis, asthma, dermatitis, psoriasis, cystic fibrosis, post transplantation late and chronic solid organ rejection, multiple sclerosis, systemic lupus erythematosus, Sjogren syndrome, Hashimoto thyroiditis, polymyositis, scleroderma, Addison disease, vitiligo, pernicious anemia, glomerulonephritis, and pulmonary fibrosis, inflammatory bowel diseases, autoimmune diabetes, diabetic retinopathy, rhinitis, ischemia-reperfusion injury, post-angioplasty restenosis, chronic obstructive pulmonary diseases (COPD), Graves disease, gastrointestinal allergies, conjunctivitis, atherosclerosis, coronary artery disease, angina, cancer metastasis, and small artery disease, and preferably graft-versus-host disease, but is not limited thereto.

In the present disclosure, the term "graft-versus-host disease" refers to a disease in which lymphocytes transfused during blood stem cell transplantation attack a host (a body of a transfused individual) with a weakened immune function, causing symptoms such as fever, rash, liver dysfunction, diarrhea, and pancytopenia (a state in which white blood cells, red blood cells, and platelets are all reduced). There are two types of acute and chronic graft-versus-host diseases that occur after transplantation. The graft-versus-host disease may very rarely occur after blood transfusion, in addition to the blood stem cell transplantation, but once a graft-versus-host response occurs after transfusion, it is very fatal.

The pharmaceutical composition of the present disclosure may be administered to an "individual" that has developed or is likely to develop autoimmune disease, and the "individual" may mean all animals including humans and may be a allogeneic individual with an individual from which the immunosuppressive T cells of the present disclosure are derived.

As used herein, the term "prevention" refers to all actions that suppress the symptoms of a specific disease or delay the progression by administering the composition of the present disclosure.

As used herein, the term "treatment" refers to all actions that improve or beneficially change the symptoms of a specific disease by administering the composition of the present disclosure.

The pharmaceutical composition of the present disclosure may further include an adjuvant in addition to the active ingredient. The adjuvant may be used with any adjuvant known in the art without limitation, but further include, for example, a Freund's complete adjuvant or an incomplete adjuvant to increase the effect thereof.

The pharmaceutical composition according to the present disclosure may be prepared in the form of incorporating the active ingredient into a pharmaceutically acceptable carrier. Here, the pharmaceutically acceptable carrier includes carriers, excipients and diluents commonly used in a pharmaceutical field. The pharmaceutically acceptable carrier that may be used in the pharmaceutical composition of the present disclosure is not limited thereto, but may include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil.

The pharmaceutical composition according to the present disclosure may be formulated and used in the form of oral formulations such as powders, granules, tablets, capsules, suspensions, emulsions, syrups, aerosols, etc., external preparations, suppositories, and sterile injectable solutions according to a conventional method.

The formulations may be prepared by using diluents or excipients, such as a filler, an extender, a binder, a wetting agent, a disintegrating agent, a surfactant, etc., which are generally used. Solid formulations for oral administration include tablets, pills, powders, granules, capsules, etc., and these solid formulations may be prepared by mixing at least one or more excipients, for example, starch, calcium carbonate, sucrose, lactose, gelatin, etc. with the active ingredient. Further, lubricants such as magnesium stearate and talc may be used in addition to simple excipients. Liquid formulations for oral administration may correspond to suspensions, oral liquids, emulsions, syrups, etc., and may include various excipients, for example, a wetting agent, a sweetener, an aromatic agent, a preserving agent, etc., in addition to the commonly used diluents, such as water and liquid paraffin. Formulations for parenteral administration include sterile aqueous solutions, non-aqueous solvents, suspensions, emulsions, lyophilized agents, and suppositories. As the non-aqueous solution and the suspension, propylene glycol, polyethylene glycol, vegetable oil such as olive oil, injectable ester such as ethyl oleate, etc. may be used. As the base material of the suppository, witepsol, Tween 61, cacao butter, laurinum, glycerogelatin, etc. may be used.

The pharmaceutical composition of the present disclosure may be administered to an individual through various routes. All methods of administration may be expected, and the pharmaceutical composition may be administered by, for example, oral, intravenous, intramuscular, subcutaneous, and intraperitoneal injection.

The dose of the pharmaceutical composition according to the present disclosure is selected in consideration of the age, body weight, sex, and physical condition of the individual. It is obvious that the concentration of the active ingredient included in the pharmaceutical composition may be variously selected according to an individual, and preferably included in the pharmaceutical composition at a concentration of 0.01 to 5,000 µg/ml. When the concentration is less than 0.01 µg/ml, pharmaceutical activity may not be exhibited, and when the concentration exceeds 5,000 µg/ml, toxicity to the human body may be exhibited.

In the present disclosure, the pharmaceutical composition for preventing or treating autoimmune diseases may further include any compound or natural extract that has already been verified as safe and known to have a therapeutic effect in order to increase a therapeutic effect of autoimmune diseases, preferably graft-versus-host disease, in addition to the immunosuppressive T cells as the active ingredient. In addition, the pharmaceutical composition for preventing or treating autoimmune diseases of the present disclosure may be used together with surgical treatment of autoimmune diseases, preferably graft-versus-host disease.

Furthermore, the present disclosure provides a cell therapeutic agent composition for preventing or treating autoimmune diseases, including immunosuppressive T cells having a phenotype of CD3⁺PD1⁺TIM3⁺, prepared through the method for the mass production of immunosuppressive T cells according to the present disclosure. In addition, the immunosuppressive T cells may further have at least one phenotype selected from the group consisting of CD3⁺PD1⁺TIM3⁻ and CD3⁺PD1⁻TIM3⁻.

As used herein, the cellular therapeutic agent refers to a medicine (in US FDA regulations) used for the purposes of treatment, diagnosis, and prevention with cells and tissues prepared through isolation from the human, culture, and special manipulation, and means a medicine used for treatment, diagnosis and prevention of diseases by these cells through a series of actions, such as ex vivo proliferating and selecting living autologous, allogeneic, or xenogenic cells to restore the functions of cells or tissues, changing the biological characteristics of cells by other methods, and the like.

The cellular therapeutic agent composition of the present disclosure may be administered directly to a lesion site, and in this case, may be administered in the form of an injection. In particular, the cellular therapeutic agent composition of the present disclosure may be formulated in the form of an injection for direct administration of immunosuppressive T cells having a phenotype of CD3⁺PD1⁺TIM3⁺, prepared through the preparation method according to the present disclosure, and in this case, may further include a hydrogel so as to be suitable for the injection.

The hydrogel that may be used in the cellular therapeutic agent of the present disclosure may include hydrogels known in the art suitable for injections without limitation, and may be at least one selected from the group consisting of small intestine submucosal tissue, hyaluronic acid, carboxymethylcellulose (CMC), alginate, chitosan, polyacrylamide, poly(N-isopropylacrylamide), β-glycerophosphate, poly(ethylene oxide)poly(propylene oxide)poly(ethyleneoxide) (Pluronic), fibrin, polyethylene oxide (PEO), and a mixture of carboxymethyl cellulose (CMC) and polyethyleneimine (PEI), but is not limited thereto.

A preferable dose of the cellular therapeutic agent composition of the present disclosure varies according to the condition and weight of a patient, the degree of a disease, a drug form, and the route and period of administration, but may be properly selected by those skilled in the art. The administration may be performed once a day or performed in several divided times, and the dose is not intended to limit the scope of the present disclosure in any way.

Further, the present disclosure provides a method for preventing or treating autoimmune diseases, including administering, to an individual, immunosuppressive T cells prepared through the method for the mass production of immunosuppressive T cells according to the present disclosure.

The "individual" means a target individual who has or is likely to develop autoimmune diseases, and the "individual" may mean all animals including humans.

The autoimmune disease may be at least one disease selected from the group consisting of graft-versus-host disease, rheumatoid arthritis, asthma, dermatitis, psoriasis, cystic fibrosis, post transplantation late and chronic solid organ rejection, multiple sclerosis, systemic lupus erythematosus, Sjogren syndrome, Hashimoto thyroiditis, polymyositis, scleroderma, Addison disease, vitiligo, pernicious anemia, glomerulonephritis, and pulmonary fibrosis, inflammatory bowel diseases, autoimmune diabetes, diabetic retinopathy, rhinitis, ischemia-reperfusion injury, post-angioplasty restenosis, chronic obstructive pulmonary diseases (COPD), Graves disease, gastrointestinal allergies, conjunctivitis, atherosclerosis, coronary artery disease, angina, cancer metastasis, and small artery disease, and preferably graft-versus-host disease, but is not limited thereto.

Hereinafter, the present disclosure will be described in more detail through Examples. These Examples are to explain the present disclosure in more detail, and it will be apparent to those skilled in the art that the scope of the present disclosure is not limited to these Examples.

### <Example 1> Cell collection and proliferation method

G-CSF (neutrogin, 10 µg/kg) was injected subcutaneously to normal peripheral blood stem cell (PBSC) donors for 5 days, and then G-CSF mobilized peripheral blood stem cells (G-PBSC) were collected using a COBE spectra cell isolator. CD3⁺lymphocytes were isolated from the collected G-PBSC by using magnetic-activated cell sorting (MACS) using CD3 Dynabeads^{™}. The isolated CD3⁺ lymphocytes were cultured in 5% human serum albumin (HSA) at a concentration of 5 × 10⁵ cells/mL for 2 or 4 days. The culture medium used was an RPMI 1640 medium containing 2 mM L-glutamine, 25 mM hydroxyethyl piperazine ethane sulfonicacid (HEPES), 1 mM sodium pyruvate, and 50 µM mercaptoethanol.

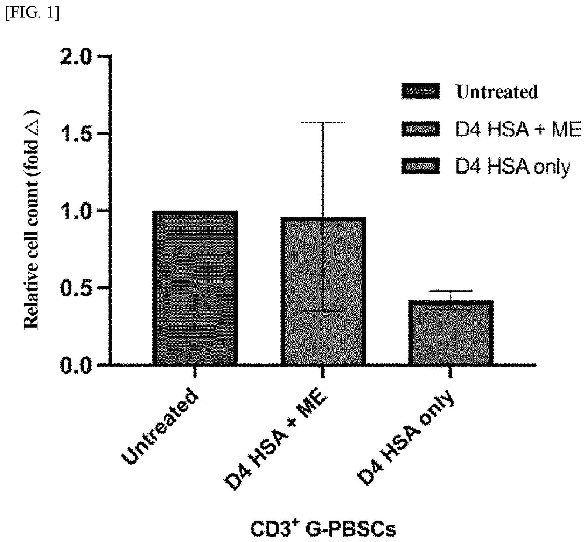

When CD3⁺G-PBSC lymphocytes were cultured for 4 days in a medium containing HSA and mercaptoethanol, the total cell number increased 1.0 ± 0.6 times compared to before culture, and when cultured with HSA alone, the cell number increased 0.4 ± 0.1 times (see FIG. 1). Through the experiment, it was confirmed that the yield of CD3⁺ lymphocytes could be increased by culturing CD3⁺ lymphocytes with HSA and mercaptoethanol.

### <Example 2> Confirmation of phenotype of proliferated cells

To confirm the expression of co-inhibitory molecules PD1, TIM3, LAG3, and TIGIT in the cells cultured in Example 1, flow cytometry using a fluorescence-activated cell sorter (FACS) was performed. At this time, the expressions of PD1, TIM3, LAG3, and TIGIT in the peripheral blood of a healthy individual (normal individual) as a control group were also confirmed.

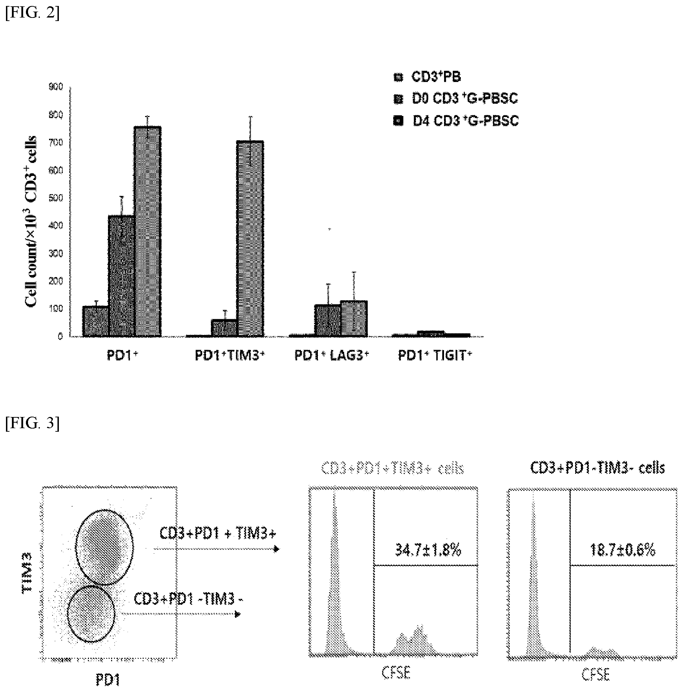

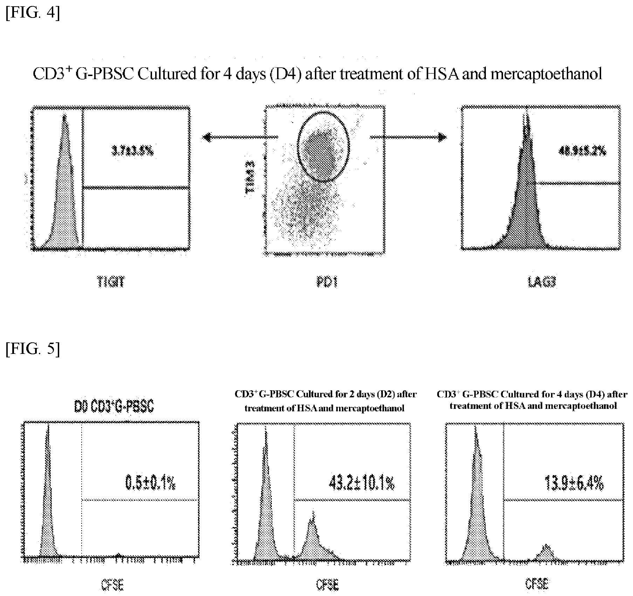

As a result, in the peripheral blood of the normal individual, CD3⁺PDI⁺TIM3⁺, CD3⁺PD1⁺LAG3⁺, and CD3⁺PD1⁺TIGIT⁺ lymphocytes were measured to be very low at 2 ± 1, 3 ± 1, and 3 ± 1 per 1 × 10³ CD3⁺G-PBSC cells, respectively. On the other hand, as a result of collecting G-PBSCs and isolating CD3+ lymphocytes after subcutaneously injecting G-CSF for 5 days, it was confirmed that CD3⁺PD1⁺TIM3⁺, CD3⁺PD1⁺LAG3⁺, and CD3⁺PD1⁺TIGIT⁺ lymphocytes increased to 68 ± 8, 105 ± 18, and 38 ± 6 per 1 × 10³ CD3⁺G-PBSCs, respectively. In addition, when the collected CD3⁺G-PBSCs were cultured in 5% HSA for 4 days, the numbers of CD3⁺PD1⁺LAG3⁺ and CD3⁺PD1⁺TIGIT⁺ lymphocytes per 1 × 10³ CD3⁺G-PBSCs were measured as 865 ± 89, 295 ± 36, or 5 ± 4, respectively. As a result, it was confirmed that the number of CD3⁺PD1⁺TIM3⁺ lymphocytes increased 433 times compared to the peripheral blood and approximately 13 times compared to before culture by the method for culturing G-PBSCs collected after subcutaneous injection of G-CSF for 5 days in a medium containing 5% HSA and 50 µM mercaptoethanol (see FIG. 2). In addition, the cultured CD3⁺PD1⁺TIM3⁺ lymphocytes expressed a LAG3 surface antigen in 48.9 ± 5.2% of total cells, and TIGIT was hardly expressed (see FIG. 4).

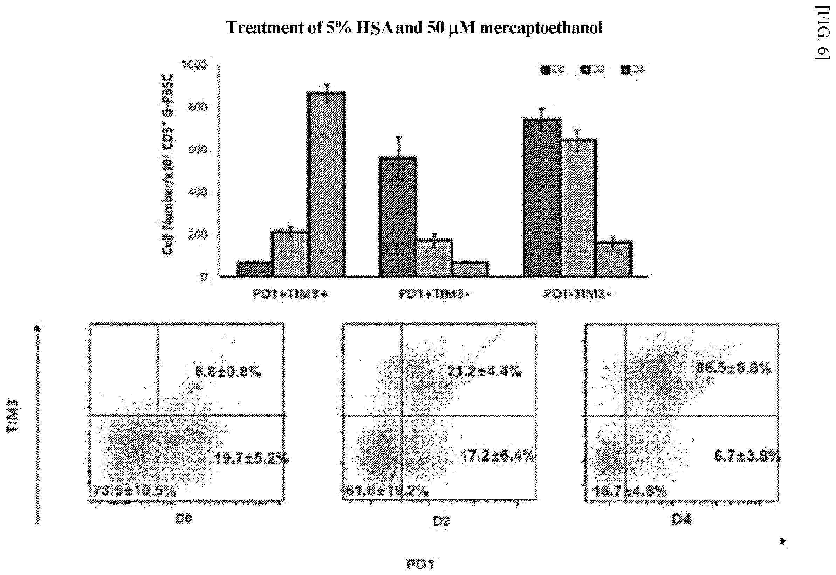

In particular, CD3⁺G-PBSC cultured for 4 days in the medium containing 5% HSA and 50 µM mercaptoethanol differentiated into two groups of lymphocytes expressing CD3⁺PD1⁺TIM3⁺ and CD3⁺PD1⁻TIM3⁻ surface antigens (see FIGS. 3 and 6). In contrast, CD3⁺G-PBSC cultured for 2 days in the medium containing 5% HSA and 50 µM mercaptoethanol differentiated into three groups of lymphocytes expressing CD3⁺PD1⁺TIM3⁺, CD3⁺PD1⁺TIM3⁻, and CD3⁺PD1⁻TIM3⁻ surface antigens (see FIG. 6).

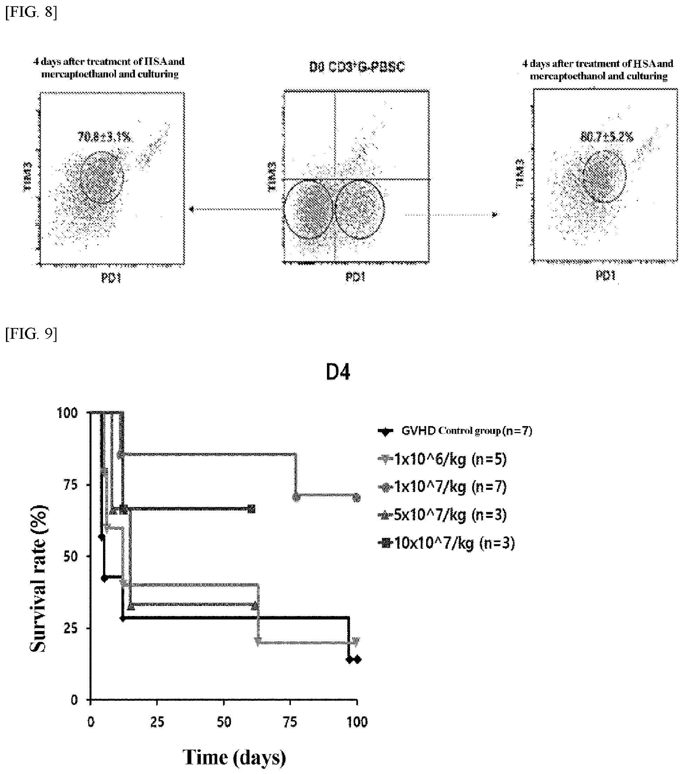

Among CD3⁺G-PBSCs cultured in the medium containing 5% HSA and 50 µM mercaptoethanol, the number of CD3⁺PD1⁺TIM3⁺ cells increased as the culture time elapsed. At this time, the number of CD3⁺PD1⁺TIM3⁺ cells before culture was 68 ± 8 per 1 × 10³ CD3⁺G-PBSCs, but the number of CD3⁺PD1⁺TIM3⁺ cells increased to 212 ± 44 after 2 days of culture, and increased to 865 ± 88 after 4 days of culture (see FIG. 6). On the other hand, the number of CD3⁺PD1⁺TIM3⁻ lymphocytes was 197 ± 52 per 1 × 10³ CD3⁺G-PBSCs before culture, but decreased to 172 ± 64 after 2 days of culture, and after 4 days of culture, almost all cells were lost (see FIG. 6). In addition, the number of CD3⁺PD1⁻TIM3⁻ lymphocytes was also decreased to 735 ± 105 per 1 × 10³ CD3⁺G-PBSCs before culture as the culture time elapsed, but decreased to 616 ± 192 after 2 days of culture, and to 67 ± 38 after 4 days of culture (see FIG. 6). According to the results, it can be estimated that CD3⁺PD1⁻TIM3⁻ lymphocytes and CD3⁺PD1⁺TIM3⁻ lymphocytes differentiate into CD3⁺PD1⁺TIM3⁺ lymphocytes as the culture time elapses. To verify the results, CD3⁺PD1⁻TIM3⁻ and CD3⁺PD1⁺TIM3⁻ lymphocytes were isolated from CD3⁺G-PBSC before culture and cultured for 4 days. As a result, after 4 days of culture, it was confirmed that 70.8 ± 3.1% of CD3⁺PD1⁻TIM3⁻ lymphocytes and 80.7 ± 5.2% of CD3⁺PD1⁺TIM3⁻ lymphocytes differentiated into CD3⁺PD1⁺TIM3⁺ lymphocytes (see FIG. 8).

### <Example 3> Confirmation of immune response suppression effect of cultured lymphocytes using mixed lymphocyte reaction

An immune response suppression effect of cells cultured using the method according to the present disclosure was confirmed using a mixed lymphocyte reaction (MLR). The mixed lymphocyte reaction is an *in vitro* experimental method for measuring the immune response of T lymphocytes to alloantigens. Specifically, when T lymphocytes are mixed and cultured with monocytes (CD14⁺ cells) from another individual, MHC between the two cells does not match, so that the T lymphocytes during culturing recognize the monocytes from another individual as alloantigens and proliferate. At this time, the immune response of T lymphocytes to alloantigens is measured by measuring the degree of proliferation of T lymphocytes. To prevent proliferation of allogeneic monocytes other than T lymphocytes, the allogeneic monocytes are irradiated. At this time, the degree of proliferation of T lymphocytes is measured by staining the T lymphocytes with 5,6-carboxyfluorescein diacetate succinimidyl ester (CFSE). The stained CFSE penetrates the cell membrane and remains by non-specifically binding to proteins within the cells, and the fluorescence amount of CFSE decreases by half each time the cells divide. Using this, the degree of lymphocyte proliferation may be confirmed by measuring the CFSE fluorescence within the cells after culture. Based on this, T lymphocytes cultured according to the method of the present disclosure for the mixed lymphocyte reaction were isolated into CD3⁺PD1⁺TIM3⁺, CD3⁺PD1⁻TIM3⁻ and CD3⁺PD1⁺TIM3⁻ lymphocytes, and the isolated lymphocytes were mixed with allogeneic monocytes (CD14⁺ cells) at a cell number ratio of 1:2 and cultured for 3 days. Thereafter, the whole cultured cells were harvested and the CSFE fluorescence level was measured, and the fraction of lymphocytes that did not proliferate in response to the alloantigen was measured as %. Anti-PD1 mAb and anti-Tim3 mAb were used in a 5% HSA cell culture medium at concentrations of 20 µg/mL and 40 µg/mL, respectively.

CD3⁺G-PBSC cultured for 4 days in the medium containing 5% HSA and 50 µM mercaptoethanol differentiated into two groups of lymphocytes of CD3⁺PD1⁺TIM3⁺ and CD3⁺PD1⁻TIM3⁻ (see FIG. 3). When a mixed lymphocyte reaction was performed on CD3⁺PD1⁺TIM3⁺ and CD3⁺PD1⁻TIM3⁻ lymphocytes, in the case of CD3⁺PD1⁺TIM3⁺ lymphocytes, 34.7 ± 1.8% of T cells did not proliferate against allogeneic monocytes, and in the case of CD3⁺PD1⁻TIM3⁻ lymphocytes, 18.7 ± 0.6% of T cells did not proliferate (see FIG. 3). Through this, it was confirmed that among the lymphocytes differentiated from CD3⁺G-PBSC, CD3⁺PD1⁺TIM3⁺ lymphocytes had a high immune response suppression effect.

In addition, to determine the immune response suppression effect of CD3⁺G-PBSC cells according to a culture time, a mixed lymphocyte reaction was performed on CD3⁺G-PBSC cells cultured for 2 or 4 days in a medium containing 5% HSA and 50 µM mercaptoethanol. As a result of the analysis, in the case of a control group, CD3⁺G-PBSC before culture, it was measured that 0.5 ± 0.1% of T cells did not proliferate against allogeneic monocytes, but in the case of CD3⁺G-PBSC cultured for 2 days in the medium containing 5% HSA and 50 µM mercaptoethanol, 43.2 ± 10.1% of T cells did not proliferate against allogeneic monocytes, and in the case of CD3⁺G-PBSC cultured for 4 days in the medium containing 5% HSA and 50 µM mercaptoethanol, 13.9 ± 6.4% of T cells did not proliferate against allogeneic monocytes (see FIG. 5). As a result, it was confirmed that CD3⁺G-PBSC cultured for 2 days had the best immunosuppressive effect (see FIG. 5), and the immunosuppressive effect of these cells was higher than that of CD3⁺PD1⁺TIM3⁺ lymphocytes isolated after 4 days of culture (see FIG. 3).

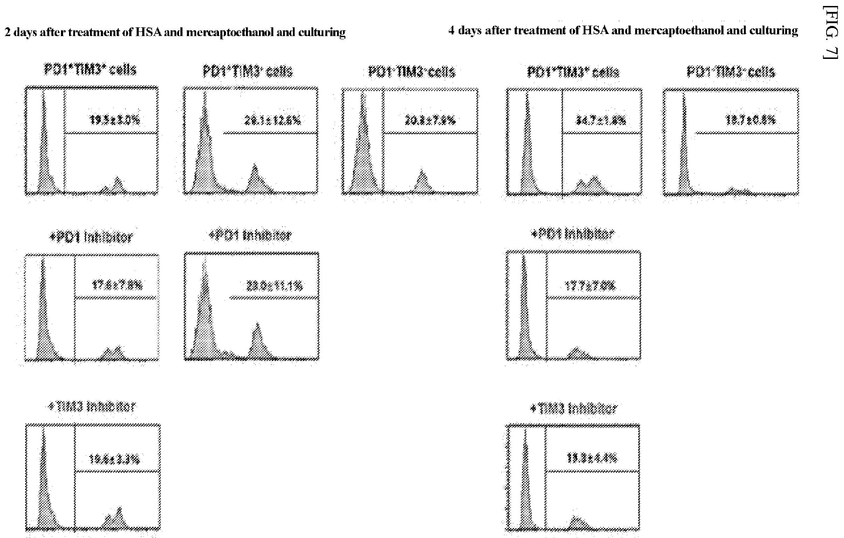

In addition, CD3⁺G-PBSC cultured for 2 days differentiated into three groups of CD3⁺PD1⁺TIM3⁺, CD3⁺PD1⁻TIM3⁻, and CD3⁺PD1⁺TIM3⁻ lymphocytes (see FIG. 6). After isolating the three groups of lymphocytes and performing a mixed lymphocyte reaction for each group, it was confirmed that in the case of CD3⁺PD1⁺TIM3⁺ lymphocytes, 19.5 ± 3.0% of the T cells did not proliferate against allogeneic monocytes, and 29.1 ± 12.6% of the T lymphocytes in the CD3⁺PD1⁺TIM3⁻ lymphocytes, and 20.3 ± 7.9% of the T lymphocytes in the CD3⁺PD1⁻TIM3⁻ lymphocytes did not proliferate against allogeneic monocytes (see FIG. 7). Compared with the result that 43.2 ± 10.1% of T lymphocytes among CD3⁺G-PBSC cultured for 2 days in the mixed lymphocyte reaction did not proliferate against allogeneic monocytes (see FIG. 5), the immunosuppressive capacity of the isolated CD3⁺PD1⁺TIM3⁺, CD3⁺PD1⁺TIM3⁻, and CD3⁺PD1⁻TIM3⁻ lymphocytes was significantly reduced (see FIG. 7). In the results, it was concluded that the method of using the entire CD3⁺G-PBSC cultured for 2 days using the method of the present disclosure was the most effective for suppressing the immune response.

In addition, the number of CD3⁺PD1⁺TIM3⁺ lymphocytes was confirmed as 86.5 ± 8.8% in CD3⁺G-PBSC cultured for 4 days, and although increased compared to 21.2 ± 4.4% after 2 days of culture, it was confirmed that the immunosuppressive effect of CD3⁺G-PBSC cultured for 4 days was significantly reduced (see FIGS. 5 and 6). There was suggested the possibility that CD3⁺PD1⁺TIM3⁺ lymphocytes after 2 and 4 days of culture may be cells expressing the same surface antigen with different immunosuppressive abilities. To verify the possibility, CD3⁺PD1⁺TIM3⁺ cells were isolated after 2 and 4 days of culture, and treated with monoclonal antibody (anti-PD1 mAb) against PD1 and monoclonal antibody (anti-Tim3 mAb) against Tim3, respectively, and then a mixed lymphocyte reaction was performed. In general, when treated with anti-PD1 mAb or anti-Tim3 mAb, it is expected to offset an alloimmune response suppression effect of immunosuppressive T lymphocytes, thereby decreasing T lymphocytes that do not respond to allogeneic monocytes. As such, as a result of treating CD3⁺PD1⁺TIM3⁺ lymphocytes isolated after 4 days of culture with anti-PD1 mAb and anti-Tim-3 mAb, respectively, it was confirmed that the T lymphocytes that did not proliferate against allogeneic monocytes decreased from 34.7 ± 1.8% to 17.7 ± 7.0% and from 34.7 ± 1.8% to 15.3 ± 4.4%, respectively, so that the alloimmune response suppression effect was offset by anti-PD1 mAb and anti-Tim-3 mAb. On the other hand, when CD3⁺PD1⁺TIM3⁺ isolated after 2 days of culture were treated with anti-PD1 mAb and anti-Tim-3 mAb, respectively, the T lymphocytes that did not proliferate against allogeneic monocytes were measured from 19.5 ± 3.0% to 17.6 ± 7.8%, and from 19.5 ± 3.0% to 19.6 ± 3.3% and 19.7 ± 3.6%, respectively, so that the expected effect of increasing the immune response due to anti-PD1 mAb and anti-Tim-3 mAb was not observed. In addition, when CD3⁺PD1⁺TIM3⁻ cells isolated from CD3⁺G-PBSC after 2 days of culture were treated with anti-PD1 mAb, the number of T lymphocytes that did not proliferate against allogeneic monocytes decreased from 29.1 ± 12.6% to 23.0 ± 11.1% to confirm the immune response increase effect of anti-PD1 mAb against CD3⁺PD1⁺TIM3⁻ cells (see FIG. 7). In these results, it was confirmed that unlike CD3⁺PD1⁺TIM3⁺ lymphocytes cultured for 4 days, CD3⁺PD1⁺TIM3⁺ lymphocytes cultured for 2 days were cells in which the immune response suppression effect of anti-PD1 mAb and anti-Tim3 mAb was not offset, and the two cells expressed the same expressed antigen, but had different functions.

### <Example 4> Confirmation of immune response suppression effect of cultured lymphocytes using xenograft GVHD mouse model

To confirm a therapeutic effect of cultured CD3⁺G-PBSC cells, an experiment was conducted using an immunodeficient xenograft aGVHD mouse model (NOD-scid IL-2Rγ null). NOD-scid IL-2Rγ null mice were mice with deficient T lymphocyte and B lymphocyte functions. After irradiating NOD-scid IL-2Rγ null mice with 3 Gy of radiation, aGVHD was induced by intraperitoneal injection of 3.5 ± 1.8 × 10⁷/kg PBMCs isolated from normal allogeneic individuals. Thereafter, CD3⁺G-PBSC lymphocytes cultured according to the method of the present disclosure were gradually increased (control group vs. 1 × 10⁶ cells/kg vs. 1 × 10⁷ cells/kg vs. 5 × 10⁷ cells/kg vs. 10 × 10⁷ cells/kg), injected into the tail veins of mice, and then the survival of the mice was confirmed and thus the therapeutic effect of cultured CD3⁺G-PBSC lymphocytes was evaluated.

As a result, in the control group (n = 7), only 14% of the mice survived 100 days after peripheral monocyte injection, whereas in a group (n = 5) injected with CD3⁺G-PBSC lymphocytes cultured for 4 days in a medium containing 5% HSA and 50 µM mercaptoethanol at a dose of 1 × 10⁶ cells/kg, 20% of the target mice survived. On the other hand, in a group injected with a dose of 1 × 10⁷ cells/kg (n = 7), 71% of the target mice survived, and thus it was confirmed that 1 × 10⁷ cells/kg of CD3⁺G-PBSC cultured for 4 days was an appropriate therapeutic dose (see FIG. 9). In addition, when the dose of CD3⁺G-PBSC lymphocytes cultured for 4 days was increased to 5 × 10⁷ cells/kg (n = 3), which was a 5-fold dose, or 10 × 10⁷ cells/kg (n = 3), which was a 10-fold dose, most of the target mice died within 20 days (see FIG. 9).

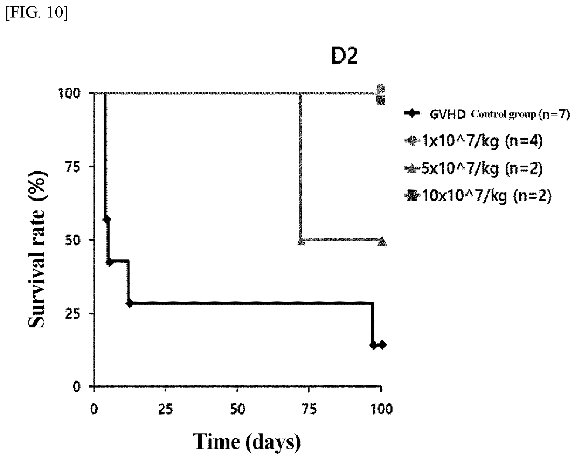

The therapeutic effect on aGVHD was evaluated by injecting CD3⁺G-PBSC cultured for 2 days in the same manner (see FIG. 10). In a group injected with a dose of 1 × 10⁷ cells/kg (n = 4), it was confirmed that 100% of the target mice survived 80 days after cell injection (see FIG. 10). In addition, it was confirmed that when CD3⁺G-PBSC lymphocytes cultured for 2 days were injected at 5 × 10⁷ cells/kg (n = 2), which was a 5-fold dose of 1 × 10⁷ cells/kg, 50% of the target mice survived, and in a group injected with 10 × 10⁷ cells/kg (n = 2), which was a 10-fold dose, all of the target mice survived without dying (see FIG. 10). Through this, it was confirmed that CD3⁺G-PBSC lymphocytes cultured with HSA effectively suppressed immune responses in vivo, and it was confirmed that the method of administering CD3⁺G-PBSC lymphocytes cultured for 2 days at a dose of 1 × 10⁷ cells/kg could most effectively suppress graft-versus-host responses.

From the results of Examples above, it was confirmed that when CD3⁺G-PBSCs were cultured for 4 days in a medium containing HSA and mercaptoethanol, most of the cells (86.5 ± 8.8%) differentiated into CD3⁺PD1⁺TIM3⁺ lymphocytes, and the differentiated CD3⁺PD1⁺TIM3⁺ lymphocytes had an excellent immune response suppression effect. In addition, CD3⁺G-PBSC cultured in HSA for 2 days included CD3⁺PD1⁺TIM3⁻ lymphocytes and CD3⁺PD1⁺TIM3⁺ lymphocytes in large quantities and showed a superior immunosuppressive effect to CD3⁺PD1⁺TIM3⁺ lymphocytes isolated after culturing for 4 days. Therefore, CD3⁺G-PBSC cultured for 2 days based on the method according to the present disclosure is the most ideal cellular therapeutic agent and is expected to be useful in the treatment of immune diseases such as graft-versus-host disease in the future.

## Claims

1. A method for the mass production of immunosuppressive T cells, comprising the following steps:
sorting CD3⁺ cells from a sample isolated from an individual; and
culturing the CD3⁺ cells in a medium containing human serum albumin (HSA).

2. The method for the mass production of immunosuppressive T cells of claim 1, wherein the sample is at least one selected from the group consisting of blood, tissue, cells, plasma, feces, urine, and semen.

3. The method for the mass production of immunosuppressive T cells of claim 1, wherein the sample is at least one selected from the group consisting of blood and peripheral blood stem cells (PBSCs).

4. The method for the mass production of immunosuppressive T cells of claim 1, wherein the individual is a healthy allogeneic individual.

5. The method for the mass production of immunosuppressive T cells of claim 1, wherein in the individual, CD3⁺ cells are mobilized into peripheral blood.

6. The method for the mass production of immunosuppressive T cells of claim 1, wherein the individual is an individual administered with a leucocyte growth factor.

7. The method for the mass production of immunosuppressive T cells of claim 6, wherein the leucocyte growth factor is a granulocyte colony stimulating factor (G-CSF).

8. The method for the mass production of immunosuppressive T cells of claim 1, wherein the medium further comprises mercaptoethanol.

9. The method for the mass production of immunosuppressive T cells of claim 8, wherein the concentration of the mercaptoethanol is 1 µM to 200 µM.

10. The method for the mass production of immunosuppressive T cells of claim 1, wherein the concentration of human serum albumin (HSA) is 1 wt% to 10 wt%.

11. The method for the mass production of immunosuppressive T cells of claim 1, wherein the culturing is performed for 1 to 7 days.

12. The method for the mass production of immunosuppressive T cells of claim 1, wherein the immunosuppressive T cells have a phenotype of CD3⁺PD1⁺TIM3⁺.

13. The method for the mass production of immunosuppressive T cells of claim 1, wherein when the culturing is performed for 1 to 3 days, the immunosuppressive T cells have at least one phenotype selected from the group consisting of CD3⁺PD1⁺TIM3⁺, CD3⁺PD1⁺TIM3⁻, and CD3⁺PD1⁻TIM3⁻.

14. A method for the mass production of immunosuppressive T cells, comprising the following steps:
sorting CD3⁺ cells from a sample isolated from an individual administered with a granulocyte colony stimulating factor (G-CSF); and
culturing the CD3⁺ cells in a medium containing human serum albumin (HSA) and mercaptoethanol for 1 to 7 days.

15. A method for inducing T cells *in vitro* into T cells having at least one phenotype selected from the group consisting of CD3⁺PD1⁺TIM3⁺, CD3⁺PD1⁺TIM3⁻ and CD3⁺PD1⁻ TIM3⁻, the method comprising the following steps:
sorting CD3⁺ cells from a sample isolated from an individual administered with a granulocyte colony stimulating factor (G-CSF); and
culturing the CD3⁺ cells in a medium containing human serum albumin (HSA) and mercaptoethanol for 1 to 3 days.

16. A method for inducing T cells *in vitro* into T cells having a phenotype of CD3⁺PD1⁺TIM3⁺, the method comprising the following steps:
sorting CD3⁺ cells from a sample isolated from an individual administered with a granulocyte colony stimulating factor (G-CSF); and
culturing the CD3⁺ cells in a medium containing human serum albumin (HSA) and mercaptoethanol for 4 to 7 days.

17. The method for the mass production of immunosuppressive T cells of claim 14, wherein the granulocyte colony stimulating factor (G-CSF) is administered at a concentration of 1 to 50 µg/kg.

18. A medium composition for the mass production of immunosuppressive T cells, comprising human serum albumin (HSA).

19. The medium composition for the mass production of immunosuppressive T cells of claim 18, further comprising:
mercaptoethanol.

20. The medium composition for the mass production of immunosuppressive T cells of claim 19, wherein the concentration of the mercaptoethanol is 1 µM to 200 µM.

21. The medium composition for the mass production of immunosuppressive T cells of claim 18, wherein the concentration of human serum albumin (HSA) is 1 wt% to 10 wt%.

22. The medium composition for the mass production of immunosuppressive T cells of claim 18, wherein the medium composition is used for culturing blood or peripheral blood stem cells (PBSCs).

23. The medium composition for the mass production of immunosuppressive T cells of claim 18, wherein the immunosuppressive T cells have a phenotype of CD3⁺PD1⁺TIM3⁺.

24. A kit for the mass production of immunosuppressive T cells, comprising a granulocyte colony stimulating factor (G-CSF) and human serum albumin (HSA).

25. The kit for the mass production of immunosuppressive T cells of claim 24, further comprising:
mercaptoethanol.

26. A medium composition for the proliferation of immunosuppressive T cells having at least one phenotype selected from the group consisting of CD3⁺PD1⁺TIM3⁺, CD3⁺PD1⁺TIM3⁻, and CD3⁺PD1⁻TIM3⁻, the medium composition comprising human serum albumin (HSA) and
**characterized by** culturing peripheral blood stem cells (PBSCs) for 1 to 3 days.

27. The medium composition of claim 26, further comprising:
mercaptoethanol.

28. A medium composition for the proliferation of immunosuppressive T cells having a phenotype of CD3⁺PD1⁺TIM3⁺, the medium composition comprising human serum albumin (HSA) and
**characterized by** culturing peripheral blood stem cells (PBSCs) for 4 to 7 days.

29. The medium composition of claim 28, further comprising:
mercaptoethanol.

30. A kit for the proliferation of immunosuppressive T cells having at least one phenotype selected from the group consisting of CD3⁺PD1⁺TIM3⁺, CD3⁺PD1⁺TIM3⁻, and CD3⁺PD1⁻TIM3⁻, the kit comprising a granulocyte colony stimulating factor (G-CSF) and human serum albumin (HSA), and
**characterized by** culturing peripheral blood stem cells (PBSCs) for 1 to 3 days.

31. The kit of claim 30, further comprising:
mercaptoethanol.

32. A kit for the proliferation of immunosuppressive T cells having a phenotype of CD3⁺PDI⁺TIM3⁺, the kit comprising a granulocyte colony stimulating factor (G-CSF) and human serum albumin (HSA), and
**characterized by** culturing peripheral blood stem cells (PBSCs) for 4 to 7 days.

33. The kit of claim 32, further comprising:
mercaptoethanol.

34. A pharmaceutical composition for preventing or treating autoimmune diseases, comprising immunosuppressive T cells prepared through the method for the mass production of immunosuppressive T cells according to claim 1.

35. The pharmaceutical composition for preventing or treating autoimmune diseases of claim 34, wherein the autoimmune disease is at least one disease selected from the group consisting of graft-versus-host disease, rheumatoid arthritis, asthma, dermatitis, psoriasis, cystic fibrosis, post transplantation late and chronic solid organ rejection, multiple sclerosis, systemic lupus erythematosus, Sjogren syndrome, Hashimoto thyroiditis, polymyositis, scleroderma, Addison disease, vitiligo, pernicious anemia, glomerulonephritis, and pulmonary fibrosis, inflammatory bowel diseases, autoimmune diabetes, diabetic retinopathy, rhinitis, ischemia-reperfusion injury, post-angioplasty restenosis, chronic obstructive pulmonary diseases (COPD), Graves disease, gastrointestinal allergies, conjunctivitis, atherosclerosis, coronary artery disease, angina, cancer metastasis, and small artery disease.

36. The pharmaceutical composition for preventing or treating autoimmune diseases of claim 34, wherein the immunosuppressive T cells have a phenotype of CD3⁺PD1⁺TIM3⁺.

37. The pharmaceutical composition for preventing or treating autoimmune diseases of claim 34, wherein the immunosuppressive T cells further have at least one phenotype selected from the group consisting of CD3⁺PD1⁺TIM3⁻ and CD3⁺PDI⁻TIM3⁻.

38. A cellular therapeutic agent composition for preventing or treating autoimmune diseases, comprising immunosuppressive T cells having a phenotype of CD3⁺PD1⁺TIM3⁺ prepared through the method for the mass production of immunosuppressive T cells according to claim 1.

39. The cellular therapeutic agent composition for preventing or treating autoimmune diseases of claim 38, wherein the immunosuppressive T cells further have at least one phenotype selected from the group consisting of CD3⁺PD1⁺TIM3⁻ and CD3⁺PDI⁻TIM3⁻.

40. A method for preventing or treating autoimmune diseases, comprising administering, to an individual, immunosuppressive T cells prepared through the method for the mass production of immunosuppressive T cells according to claim 1.

41. The method for preventing or treating autoimmune diseases of claim 40, wherein the autoimmune disease is at least one disease selected from the group consisting of graft-versus-host disease, rheumatoid arthritis, asthma, dermatitis, psoriasis, cystic fibrosis, post transplantation late and chronic solid organ rejection, multiple sclerosis, systemic lupus erythematosus, Sjogren syndrome, Hashimoto thyroiditis, polymyositis, scleroderma, Addison disease, vitiligo, pernicious anemia, glomerulonephritis, and pulmonary fibrosis, inflammatory bowel diseases, autoimmune diabetes, diabetic retinopathy, rhinitis, ischemia-reperfusion injury, post-angioplasty restenosis, chronic obstructive pulmonary diseases (COPD), Graves disease, gastrointestinal allergies, conjunctivitis, atherosclerosis, coronary artery disease, angina, cancer metastasis, and small artery disease.
